# EUROPÄISCHE PATENTSCHRIFT

(11) **EP 1 781 298 B1**
(45) Veröffentlichungstag und Bekanntmachung des Hinweises auf die Patenterteilung: **04.01.2012**
(21) Anmeldenummer: 05739576.6
(22) Anmeldetag: 18.04.2005
(51) Int. Cl.: A61K 31/538, A61P 11/06, A61K 45/06

(54) **ARZNEINMITTELKOMBINATIONEN ENTHALTEND BENZOXAZINE ZUR BEHANDLUNG VON ATEMWEGSERKRANKUNGEN**
PHARMACEUTICAL COMBINATIONS CONTAINING BENZOXAZINE FOR TREATING RESPIRATORY DISEASES
COMBINAISONS MEDICAMENTEUSES CONTENANT DE LA BENZOXAZINE POUR TRAITER DES MALADIES DES VOIES RESPIRATOIRES

(30) Priorität: 22.04.2004 DE 102004019540; 03.11.2004 DE 102004052987; 07.02.2005 EP 05002496
(43) Veröffentlichungstag der Anmeldung: 09.05.2007
(60) Teilanmeldung: 11187038.2
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: BOUYSSOU, Thierry, 88447 Warthausen (DE); KONETZKI, Ingo, 88447 Warthausen (DE); PESTEL, Sabine, 88448 Attenweiler (DE); SCHNAPP, Andreas, 88400 Biberach (DE); HOENKE, Christoph, 55218 Ingelheim (DE); LUSTENBERGER, Philipp, 4056 Basel (CH); RUDOLF, Klaus, 88447 Warthausen (DE); PIEPER, Michael, P., 88400 Biberach (DE); PAIRET, Michel, 88400 Biberach (DE)
(74) Vertreter: Hammann, Heinz
(86) Internationale Anmeldenummer: PCT/EP2005/004073
(87) Internationale Veröffentlichungsnummer: WO 2005/102349

(56) Entgegenhaltungen:
- WO-A-01/83462
- WO-A-03/000241
- WO-A-2004/000840
- WO-A-2004/004775
- WO-A-2004/045618
- DE-A1- 10 246 374

## Beschreibung

Die vorliegende Erfindung betrifft neue Arzneimittelkombinationen, die neben einer oder mehrerer, bevorzugt einer Verbindung der Formel **1** 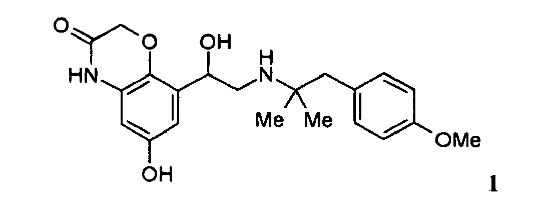 wenigstens einen weiteren Wirkstoff **2** enthalten, Verfahren zu deren Herstellung sowie deren Verwendung als Arzneimittel.

Die WO 2001/83462 offenbart weitere Verbindungen aus dieser Substanzklasse. Die Verbindung der Formel **1** wurde in WO 2004/045618 beschrieben.

### Detaillierte Beschreibung der Erfindung

Die vorliegende Erfindung betrifft Arzneimittelkombinationen, die neben einer Verbindung 1 (6-Hydroxy-8-{1-hydroxy-2-[2-(4-methoxy-phenyl)-1,1-dimethyl-ethylamino]-ethyl}-4H-benzo[1,4]oxazin-3-on) 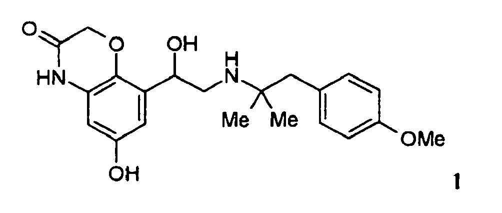 gegebenenfalls in Form der einzelnen optischen Isomeren, Mischungen der einzelnen Enantiomeren oder Racemate, der Säureadditionssalze mit pharmakologisch unbedenklichen Säuren, sowie gegebenenfalls in Form der Solvate und/oder Hydrate;
als weiteren Wirkstoff **2** ein Tiotropiumsalz **2a** oder Ciclesonid **2b** oder
als weitere Wirkstoffe **2** ein Tiotropiumsalz **2a** und Ciclesonid **2b** enthält.

In den erfindungsgemäßen Arzneimittelkombinationen kann die Verbindung der Formel **1** in Form der einzelnen optischen Isomeren, Mischungen der einzelnen Enantiomeren oder Racemate enthalten sein. Besonders bevorzugt sind hierbei diejenigen Arzneimittelkombinationen in denen die Verbindung der Formel **1** in Form der enantiomerenreinen Verbindungen, bevorzugt in Form der R-Enantiomere enthalten ist. Verfahren zur Auftrennung von Racematen in die jeweiligen Enantiomere sind im Stand der Technik bekannt und können zur Darstellung der enantiomerenreinen R- bzw. S-Enantiomere der Verbindung der Formel **1** in analoger Art und Weise zur Anwendung gelangen.

Ein weiterer Aspekt der vorliegenden Erfindung betrifft Arzneimittelkombinationen, welche die vorstehend genannte Verbindung der Formel 1 in Form der Säureadditionssalze mit pharmakologisch unbedenklichen Säuren sowie gegebenenfalls in Form der Solvate und/oder Hydrate enthalten.

Unter Säureadditionssalzen mit pharmakologisch unbedenklichen Säuren der Verbindung **1** werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Bevorzugte Arzneimittelkombinationen enthalten neben ein oder mehrerer, bevorzugt einer Verbindung der Formel **1** als weiteren Wirkstoff ein Tiotropiumsalz **2a** gegebenenfalls in Kombination mit pharmazeutisch verträglichen Hilfsstoffen.

In den vorstehend genannten Tiotropiumsalzen **2a** stellen die Kationen Tiotropium die pharmakologisch aktiven Bestandteile dar. Eine explizite Bezugnahme auf die vorstehend genannten Kationen erfolgt durch die Bezeichnungen **2a'.** Jede Bezugnahme auf die vorstehend genannten Salze **2a** schließt naturgemäß eine Bezugnahme auf die entsprechenden Kationen Tiotropium (**2a'**) mit ein.

Unter den Tiotropiumsalzen **2a** werden erfindungsgemäß diejenigen Verbindungen verstanden, die neben den Kationen Tiotropium als Gegenion (Anion) Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.
Bei den Tiotropiumsalzen sind die Methansulfonate und Bromide von besonderer Bedeutung. Von besonderer Bedeutung ist das Tiotropiumbromid.

Die vorstehend genannten Salze können in den erfindungsgemäßen Arzneimittelkombinationen gegebenenfalls in Form ihrer Solvate oder Hydrate, bevorzugt in Form ihrer Hydrate vorliegen. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Gegebenenfalls weisen die vorstehend genannten Anticholinergika chirale Kohlenstoffzentren auf. In diesem Fall können die erfindungsgemäßen Arzneimittelkombinationen die Anticholinergika in Form ihrer Enantiomere, Mischungen der Enantiomere oder Racemate enthalten, wobei vorzugsweise enantiomerenreine Anticholinergika zum Einsatz gelangen.

Erfindungsgemäß ferner bevorzugte Arzneimittelkombinationen enthalten neben einer Verbindung der Formel **1** als weiteren Wirkstoff Ciclesonid **2b,** gegebenenfalls in Form des Racemate, Enantiomere oder Diastereomere und gegebenenfalls in Form ihrer Salze und Derivate, ihrer Solvate und/oder Hydrate.

Jede Bezugnahme auf Ciclesonid **2b** schließt eine Bezugnahme auf deren gegebenenfalls existierende Salze oder Derivate, Hydrate oder Solvate mit ein. Beispiele möglicher Salze und Derivate können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Die erfindungsgemäßen Arzneimittelkombinationen aus der Verbindung der Formel **t** mit wenigstens einem weiteren Wirkstoff 2 sind nicht beschränkt auf binäre Wirkstoffkombinationen. Die vorstehend genannten Kombinationen, die neben einer Verbindung der Formel **1** einen weiteren Wirkstoff **2** enthalten, können ferner Tiotropoiumsalze **2a** und Ciclesonid **2b** enthalten.

Erfindungsgemäß von herausragender Bedeutung sind all diejenigen im Rahmen der vorliegenden Erfindung offenbarten Arzneimittelkombinationen, die die Verbindung der Formel **1** in Form Ihrer R-Enantiomere enthalten.

Im Rahmen der vorliegenden Erfindung wird unter Arzneimittelkombination der Komponenten **1** und **2** die gemeinsame Applikation beider Wirkstoffe in einer einzigen Darreichungsform bzw. Formulierung oder die getrennte Applikationen der beiden Wirkstoffe in getrennten Darreichungsformen verstanden. Werden die Wirkstoffe **1** und **2** in getrennten Darreichungsformen appliziert, kann diese getrennte Applikation gleichzeitig oder zeitlich abgestuft, das heißt nacheinander erfolgen.

Ein Aspekt der vorliegenden Erfindung betrifft vorstehend genannte Arzneimittelkombinationen, welche neben therapeutisch wirksamen Mengen von **1** und **2** einen pharmazeutisch verträglichen Trägerstoff enthalten. Ein Aspekt der vorliegenden Erfindung betrifft vorstehend genannte Arzneimittel, welche neben therapeutisch wirksamen Mengen von **1** und **2** keinen pharmazeutisch verträglichen Trägerstoff enthalten.

Die vorliegende Erfindung betrifft ferner die Verwendung therapeutisch wirksamer Mengen der Wirkstoffe **1** zur Herstellung eines ferner einen oder mehrere, bevorzugt einen Wirkstoff **2** enthaltenden Arzneimittels zur Behandlung von entzündlichen und obstruktiven Atemwegserkrankungen, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen (Antiarrhythmikum), zur Therapie des Kreislaufschocks (Gefäßerweiterung und Steigerung des Herzzeitvolumens) sowie zur Behandlung von Juckreiz und Entzündungen der Haut.

Ein bevorzugter Aspekt der vorliegenden Erfindung betrifft die Verwendung therapeutisch wirksamer Mengen der Wirkstoffe **1** zur Herstellung eines ferner einen oder mehrere, bevorzugt einen Wirkstoff **2** enthaltenden Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist vorstehend genannte Verwendung der erfindungsgemäßen Arzneimittelkombinationen zur Herstellung eines Arzneimittels zur Behandlung von obstruktiven Lungenerkrankungen, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

Bevorzugt ist ferner die vorstehend genannte Verwendung der erfindungsgemäßen Arzneimittelkombinationen zur Herstellung eines Arzneimittels zur Behandlung von Lungenemphysemen die ihren Ursprung haben in COPD oder α1-Proteinase-Inhibitor-Mangel.

Bevorzugt ist ferner die vorstehend genannte Verwendung der erfindungsgemäßen Arzneimittelkombinationen zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die vorstehend genannte Verwendung der erfindungsgemäßen Arzneimittelkombinationen zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die vorstehend genannte Verwendung der erfindungsgemäßen Arzneimittelkombinationen zur Herstellung eines Arzneimittels zur Behandlung von Zystischer Fibrose bzw. Mukoviszidose.

Bevorzugt ist ferner die vorstehend genannte Verwendung der erfindungsgemäßen Arzneimittelkombinationen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die vorstehend genannte Verwendung der erfindungsgemäßen Arzneimittelkombinationen zur Herstellung eines Arzneimittels zur Behandlung von Bronchiektasen.

Bevorzugt ist ferner die vorstehend genannte Verwendung der erfindungsgemäßen Arzneimittelkombinationen zur Herstellung eines Arzneimittels zur Behandlung von ARDS (adult respiratory distress syndrom).

Bevorzugt ist ferner die vorstehend genannte Verwendung der erfindungsgemäßen Arzneimittelkombinationen zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorstehend genannte Verwendung der erfindungsgemäßen Arzneimittelkombinationen zur Herstellung eines Arzneimittels zur Behandlung von Asthma oder COPD. Von besonderer Bedeutung ist ferner die vorstehend genannte Verwendung der erfindungsgemäßen Arzneimittelkombinationen zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Die vorliegende Erfindung betrifft ferner die Verwendung therapeutisch wirksamer Mengen eines Wirkstoffs der Formel 1 in Kombination mit therapeutisch wirksamen Mengen eines Wirkstoffs 2 zur Herstellung eines Arzneimittels zur Behandlung einer der vorstehend genannten Erkrankungen.

Die vorliegende Erfindung betrifft ferner ein Verfahren zur Behandlung einer der vorstehend genannten Erkrankungen, welches dadurch gekennzeichnet ist, dass therapeutisch wirksame Mengen eines Wirkstoffs der Formel **1** in Kombination mit therapeutisch wirksamen Mengen eines Wirkstoffs **2** appliziert werden.

Im Rahmen der erfindungsgemäßen Arzneimittelkombinationen können pro Einmalgabe beispielsweise 0,1 - 1000µg einer Verbindung der Formel 1 appliziert werden. Bevorzugt werden pro Einmalgabe 1 - 500 µg, besonders bevorzugt 3 - 100 µg der Verbindung der Formel **1** appliziert, wobei ein Dosierungsbereich von 5 - 75µg, bevorzugt von 7 - 50 µg erfindungsgemäß bevorzugt ist. Besonders bevorzugt werden die erfindungsgemäßen Arzneimittel in einer solchen Menge appliziert, dass pro Einmalgabe 9 - 40 µg, besonders bevorzugt 11 - 30µg, ferner bevorzugt 12 - 25 µg der Verbindung der Formel 1 verabreicht werden. Beispielsweise und ohne den Gegenstand der vorliegenden Erfindung darauf zu beschränken, können pro Einmalgabe 5µg, 7,5µg, 10µg, 12,5µg, 15µg, 17,5µg, 20µg, 22,5µg, 25µg, 27,5µg, 30µg, 32,5µg, 35µg, 37,5µg, 40µg, 42,5µg, 45µg, 47,5µg, 50µg, 52,5µg, 55µg, 57,5µg, 60µg, 62,5µg, 65µg, 67,5µg, 70µg, 72,5µg oder 75µg einer Verbindung der Formel **1** appliziert werden.

Die vorstehend genannten Dosierungen beziehen sich auf die Verbindung der Formel **1 in** Form ihrer freien Base. Werden die Verbindung der Formel 1 in Form ihrer pharmazeutisch verträglichen Säureadditionssalze appliziert, sind aus vorstehend genannten Dosisbereichen die entsprechenden Dosisbereiche für die Säureadditionssalze unter Berücksichtigung des Molekulargewichts der verwendeten Säuren für den Fachmann leicht berechenbar. Besonders bevorzugt, werden die Verbindung der Formel 1 bei o.g. Dosisbereichen in Form der enantiomerenreinen Verbindungen, besonders bevorzugt in Form ihrer R-Enantiomere appliziert.

Werden die Verbindung der Formel **1** in Kombination mit Tiotropiumsalzen appliziert, variiert die Menge des eingesetzten Anticholinergikums stark in Abhängigkeit von der Wahl des Wirkstoffs.

Ohne den Umfang der Erfindung darauf zu beschränken können im Falle des Tiotropiums **2a'** solche Mengen an Anticholinergikum appliziert werden, dass pro Einmalgabe 0,1 - 80µg, bevorzugt 0,5 - 60 µg, besonders bevorzugt etwa 1 - 50µg **2a'** enthalten sind. Beispielsweise und ohne den Gegenstand der vorliegenden Erfindung darauf zu beschränken, können pro Einmalgabe 2,5µg, 5µg, 10µg, 18µg, 20µg, 36µg oder 40µg **2a**' appliziert werden. Die jeweils entsprechende Menge des zum Einsatz gelangenden Salzes **2a** oder gegebenenfalls zum Einsatz gelangender Hydrate oder Solvate sind für den Fachmann je nach Wahl des Anions leicht berechenbar. Wird als erfindungsgemäß bevorzugtes Tiotropiumsalz **2a** beispielsweise Tiotropiumbromid verwendet, entsprechen die vorstehend beispielhaft genannten pro Einmalgabe applizierten Wirkstoffmengen von **2a'** den nachfolgenden pro Einmalgabe applizierten Mengen an **2a:** 3µg, 6µg, 12µg, 21,7µg, 24,1µg, 43,3µg und 48,1µg **2a**. Im Falle des Tiotropiums **2a'** erfolgt die Applikation der vorstehend genannten Dosierungen vorzugsweise ein- oder zweimal täglich, wobei die einmal tägliche Applikation erfindungsgemäß besonders bevorzugt ist.

Werden die Verbindung der Formel 1 in Kombination mit Ciclesonid **2b** appliziert, werden vorzugsweise etwa 1 - 10000 µg **2b** pro Einmalgabe appliziert. Bevorzugt werden solche Mengen an **2b** appliziert, dass pro Einmalgabe 5 - 5000µg, bevorzugt 5 - 2500 µg, besonders bevorzugt 10-1000 µg **2b** enthalten sind. Beispielsweise und ohne den Gegenstand der vorliegenden Erfindung darauf zu beschränken, können pro Einmalgabe 10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg oder 1000µg **2b** appliziert werden. Bei gegebenenfalls zum Einsatz gelangenden Salzen oder Derivaten von **2b** ist die entsprechende Menge des zum Einsatz gelangenden Salzes/Derivates für den Fachmann je nach Wahl des Salzes/Derivates aus vorstehenden Werten leicht berechenbar.

Die beiden Wirkstoffkomponenten **1** und **2** können - zusammen oder räumlich getrennt - jeweils in an sich bekannter Weise inhalativ, oral, parenteral oder auf anderem Wege in weitestgehend üblichen Formulierungen wie beispielsweise Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Geeignete Anwendungsformen zur Applikation der Verbindung der Formel **1** und **2** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilflösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wäßriger Suspensionen können die Wirkstoffe außer den oben genannten Hilfsstoffen mit verschiedenen Geschmacksautbesserern oder Farbstoffen versetzt werden.

Vorzugsweise wird auch bei getrennter Applikation der beiden Komponenten **1** und **2** zumindest die Komponente **1** inhalativ appliziert. Wird die Komponente **1** inhalativ appliziert, kann die Komponente **2** bei getrennter Gabe der beiden Wirkstoffe auch beispielsweise oral oder auch parenteral auf Basis im Stand der Technik üblicher Formulierungen wie Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Vorzugsweise werden die erfindungsgemäßen Arzneimittelkombinationen allerdings inhalativ mittels einer einzigen, beide Wirkstoffe **1** und **2** enthaltenden oder mittels getrennter, jeweils nur einen der Wirkstoffe **1** und **2** enthaltenden, für die inhalative Anwendung geeigneter Darreichungsformen appliziert.

Als inhalierbare Darreichungsformen kommen Inhalationspulver, treibgashaltige Dosieraerosole oder treibgasfreie Inhalationslösungen in Betracht. Erfindungsgemäße Inhalationspulver enthaltend die Wirkstoffkombination aus 1 und 2 können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhalationslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthaltend die erfindungsgemäßen Wirkstoffkombinationen:

Die erfindungsgemäßen Inhalationspulver können **1** und **2** entweder allein oder im Gemisch mit geeigneten physiologisch unbedenkliche Hilfsstoffen enthalten. Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose, Trehalose oder Glucose, insbesondere, aber nicht ausschließlich in Form ihrer Hydrate, bevorzugt ist.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1** und **2,** vorzugsweise mit einer mittleren Teilchengröße von 0,5 bis 10µm, besonders bevorzugt von 1 bis 6µm, dem Hilfsstoff oder der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt. Die erfindungsgemäßen Inhalationspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden.
Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Die erfindungsgemäßen Inhalationspulver, die **1** und **2** gegebenenfalls in Verbindung mit einem physiologisch verträglichen Hilfsstoff enthalten, können beispielsweise mittels des unter dem Namen Turbuhaler^{®} bekannten Inhalators beziehungsweise mit Inhalatoren wie sie beispielsweise in der EP 237507 A offenbart werden, appliziert werden. Vorzugsweise werden die erfindungsgemäßen Inhalationspulver, die neben **1** und **2** physiologisch unbedenkliche Hilfsstoffenthalten, allerdings in Kapseln abgefüllt (zu sogenannten Inhaletten), die in Inhalatoren wie beispielsweise in der WO 94/28958 beschrieben, zur Anwendung gelangen.

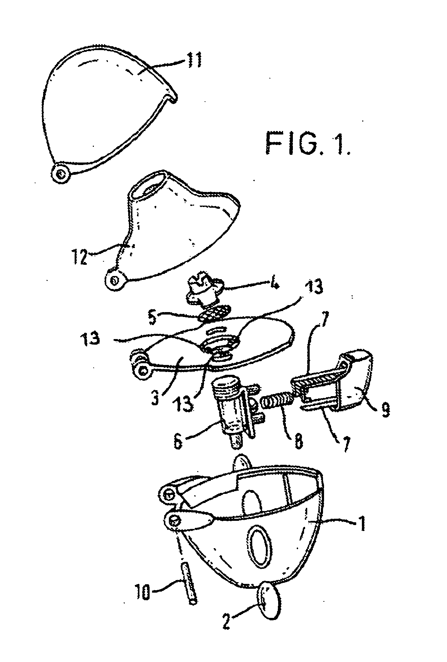

Ein zur Anwendung der erfindungsgemäßen Arzneimittelkombination in Inhaletten besonders bevorzugter Inhalator ist Figur 1 zu entnehmen.
Dieser Inhalator (Handihaler^{®}) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfindungsgemäßen Inhalationspulver im Sinne der vorstehend gennannten bevorzugten Anwendung in Kapseln abgefüllt werden, bieten sich Füllmengen von 1 bis 30mg pro Kapsel an. Diese enthalten erfindungsgemäß entweder gemeinsam oder jeweils die bereits vorstehend für 1 und 2 genannten Dosierungen pro Einmalgabe.

### B) Treibgashaltige Inhalationsaerosole enthaltend die erfindungsgemäßen Wirkstoffkombinationen:

Erfindungsgemäße treibgashaltige Inhalationsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.
Die zur Herstellung der erfindungsgemäßen Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind Halogenierte Alkanderivate ausgewählt aus TG11, TG12, TG134a (1,1,1,2-Tetrafluorethan), TG227 (1,1,1,2,3,3,3-Heptafluorpropan) und Mischungen derselben, wobei die Treibgase TG134a, TG227 und Gemische derselben bevorzugt sind.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhalationsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2.**

Liegen die Wirkstoffe **1** und/oder **2** in dispergierter Form vor weisen die Wirkstoffteilchen bevorzugt eine mittlere Teilchengröße von bis zu 10 µm, bevorzugt von 0,1 bis 6 µm, besonders bevorzugt von 1 bis 5 µm auf.

Die vorstehend genannten erfindungsgemäßen treibgashaltigen Inhalationaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, dass sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhalationsaerosolen sind aus dem Stand der Technik bekannt.

### C) Treibgasfreie Inhalationslösungen oder Suspensionen enthaltend die erfindungsgemäßen Wirkstoffkombinationen:

Erfindungsgemäß treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70 Volumenprozent, insbesondere bei bis zu 60 Volumenprozent Ethanol. Die restlichen Volumenprozente werden von Wasser aufgefüllt. Die **1** und **2** getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg / 100 ml, bevorzugt unter 50 mg/100 ml, besonders bevorzugt unter 20 mg/100 ml. Generell sind solche Inhalationslösungen bevorzugt, in denen der Gehalt an Natriumedetat bei 0 bis 10mg/100ml liegt.

Den erfindungsgemäßen treibgasfreien Inhalationslösungen können Co-Solventien und/oder weitere Hilfsstoffe zugesetzt werden.
Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhalationslösungen sind besonders solche Inhalatoren, die eine kleine Menge einer flüssigen Formulierung in der therapeutisch notwendigen Dosierung binnen weniger Sekunden in ein therapeutischinhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 10 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat^{®} bekannt.

Die vorstehend genannten Vertreter der Wirkstoffe **2** sind im Stand der Technik bekannt. Die Verbindung der Formel **1** sind dagegen noch nicht im Stand der Technik bekannt, wurden aber in WO 2004/045618 beschrieben. Die nachstehend beschriebenen Synthesebeispiele dienen der Illustration möglicher synthetischer Zugänge zu den neuen Verbindung der Formel 1. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel: 6-Hydroxy-8-{1-hydroxy-2-[2-(4-methoxy-phenyl)-1,1-dimethyl-ethylamino]-ethyl}-4H-benzo[1,4]oxazin-3-on

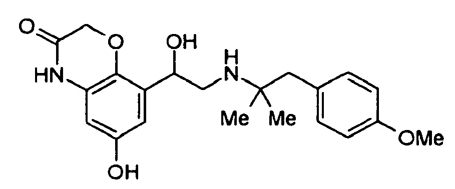

### a) 8-{2-[1,1-Dimethyl-2-(4-methoxy-phenyl)-ethylamino]-1-hydroxy-ethyl}-6-benzyloxy-4H-benzo[1,4]oxazin-3-on

Zu einer Lösung von 3.6 g 1,1-Dimethyl-2-(4-methoxyphenyl)-ethylamin in 100 mL Ethanol werden bei 70°C 7.5 g (6-Benzyloxy-4H-benzo[1,4]oxazin-3-on)-glyoxalhydrat gegeben und man läßt 15 Minuten rühren. Anschließend werden innerhalb von 30 Minuten bei 10 bis 20°C 1 g Natriumborhydrid zugesetzt. Es wird eine Stunde gerührt, mit 10 mL Aceton versetzt und über weitere 30 Minuten gerührt. Die Reaktionsmischung wird mit 150 mL Ethylacetat verdünnt, mit Wasser gewaschen, mit Natriumsulfat getrocknet und eingeengt. Der Rückstand wird in 50 mL Methanol und 100 mL Ethylacetat gelöst und mit konz. Salzsäure sauer gestellt. Nach Zugabe von 100 mL Diethylether fällt das Produkt aus. Die Kristalle werden abfiltriert, gewaschen und in 50 mL Ethanol umkristallisiert. Ausbeute: 7 g (68%; Hydrochlorid); Schmp. = 232-234°C.

### b) 8-{2-[1,1-Dimethyl-2-(4-methoxy-phenyl)-ethylaminol-1-hydroxy-ethyl}-6-hydroxy-4H-benzo[1,4]oxazin-3-on

6.8 g der vorstehend erhaltenen Benzylverbindung werden in 125 mL Methanol unter Zusatz von 1 g Palladium auf Kohle (5%ig) bei Raumtemperatur und Normaldruck hydriert. Der Katalysator wird abfiltriert und das Filtrat vom Lösungsmittel befreit. Nach Umkristallisation des Rückstandes in 50 mL Aceton und etwas Wasser wird ein Feststoff erhalten, der abfiltriert und gewaschen wird.
Ausbeute: 5.0 g (89 %; Hydrochlorid); Schmp. = 155-160°C.

Die (R)- und (S)-Enantiomere von Beispiel 1.1 können aus dem Racemat beispielsweise mittels chiraler HPLC erhalten werden (z.B. Säule: Chirobiotic T der Firma Astec, 250 x 22.1 mm, 5 µm). Als mobile Phase kann Methanol mit 0.05 % Triethylamin und 0.05% Essigsäure verwendet werden. Die Retentionszeiten des R- bzw. S-Enantiomers liegen je nach Fluß zwischen 35 und 65 min, wobei das R-Enantiomer zuerst eluiert. Erfindungsgemäß von herausragender Bedeutung ist das R-Enantiomer des Beispiels 1.

## Patentansprüche

1. Arzneimittelkombinationen, die neben einer Verbindung **1** (6-Hydroxy-8-{1-hydroxy-2-[2-(4-methoxy-phenyl)-1,1-dimethyl-ethylamino]-ethyl}-4H-benzo[1,4]oxazin-3-on) 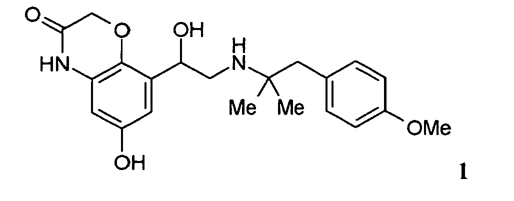 gegebenenfalls in Form der einzelnen optischen Isomeren, Mischungen der einzelnen Enantiomeren oder Racemate, der Säureadditionssalze mit pharmakologisch unbedenklichen Säuren sowie gegebenenfalls in Form der Solvate und/oder Hydrate;
als weiteren Wirkstoff **2** ein Tiotropiumsalz **2a** oder Ciclesonid **2b** enthalten.

2. Arzneimittelkombinationen nach Anspruch 1, die Verbindung 1 in Form der einzelnen optischen Isomeren, Mischungen der einzelnen Enantiomeren oder Racemate enthalten.

3. Arzneimittelkombinationen nach einem der Ansprüche 1 oder 2, die die Verbindung 1 in Form der Säureadditionssalze mit pharmakologisch unbedenklichen Säuren sowie gegebenenfalls in Form der Solvate und/oder Hydrate enthalten.

4. Arzneimittelkombinationen nach einem der Ansprüche 1 bis 3, die die Verbindung 2a in Form des Bromids, gegebenenfalls in Form der Solvate und/oder Hydrate enthalten.

5. Arzneimittelkombinationen nach einem der Ansprüche 1 bis 4, **dadurch gekennzeichnet, dass** sie neben therapeutisch wirksamen Mengen von **1** und **2** einen pharmazeutisch verträglichen Trägerstoff enthalten.

6. Arzneimittelkombinationen nach einem der Ansprüche 1 bis 4, **dadurch gekennzeichnet, dass** sie neben therapeutisch wirksamen Mengen von **1** und **2** keinen pharmazeutisch verträglichen Trägerstoff enthalten.

7. Arzneimittelkombination nach einem der Ansprüche 1 bis 6, **dadurch gekennzeichnet, dass** sie in Form einer für die Inhalation geeigneten Darreichungsform vorliegt.

8. Arzneimittelkombination nach Anspruch 7, **dadurch gekennzeichnet, dass** es sich um eine Darreichungsform ausgewählt aus der Gruppe Inhalationspulver, treibgashaltige Dosieraerosole und treibgasfreie Inhalationslösungen oder -suspensionen handelt.

9. Arzneimittelkombination nach Anspruch 8, **dadurch gekennzeichnet, dass** die Darreichungsform ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinander enthält.

10. Arzneimittelkombination nach Anspruch 8, **dadurch gekennzeichnet, dass** die Darreichungsform ein treibgashaltiges Inhalationsaerosol ist, welches **1** und **2** in gelöster oder dispergierter Form enthält.

11. Arzneimittelkombination nach Anspruch 10, **dadurch gekennzeichnet, dass** das Inhalationsaerosol als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

12. Arzneimittelkombination nach Anspruch 11, **dadurch gekennzeichnet, dass** das Treibgas TG11, TG12, TG134a, TG227 oder Gemische davon, bevorzugt TG134a, TG227 oder ein Gemisch davon darstellt.

13. Arzneimittelkombination nach Anspruch 8, **dadurch gekennzeichnet, dass** es sich bei der Darreichungsform um eine treibgasfreie Inhalationslösung oder -suspension handelt, die als Lösemittel Wasser, Ethanol oder ein Gemisch aus Wasser und Ethanol enthält.

14. Verwendung einer Arzneimittelkombination nach einem der Ansprüche 1 bis 11 zur Herstellung eines Arzneimittels zur Behandlung von entzündlichen und obstruktiven Atemwegserkrankungen, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen (Antiarrhythmikum), zur Therapie des Kreislaufschocks (Gefäßerweiterung und Steigerung des Herzzeitvolumens) sowie zur Behandlung von Juckreiz und Entzündungen der Haut.

15. Verwendung nach Anspruch 14, zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

16. Verwendung nach Anspruch 15, zur Herstellung eines Arzneimittels zur Behandlung von obstruktiven Lungenerkrankungen die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung).

17. Verwendung nach Anspruch 15, zur Herstellung eines Arzneimittels zur Behandlung von Lungenemphysemen, die ihren Ursprung haben in COPD oder α1-Proteinase-Inhibitor-Mangel.

18. Verwendung nach Anspruch 15, zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

19. Verwendung nach Anspruch 15, zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

20. Verwendung nach Anspruch 15, zur Herstellung eines Arzneimittels zur Behandlung von Zystischer Fibrose bzw. Mukoviszidose.

21. Verwendung nach Anspruch 15, zur Herstellung eines Arzneimittels zur Behandlung von Bronchitiden, wie Bronchitis aufgrund bakterieller oder viraler Infektion, Allergische Bronchitis und Toxische Bronchitis.

22. Verwendung nach Anspruch 15, zur Herstellung eines Arzneimittels zur Behandlung von Bronchiektasen.

23. Verwendung nach Anspruch 15, zur Herstellung eines Arzneimittels zur Behandlung von ARDS (adult respiratory distress syndrom).

24. Verwendung nach Anspruch 15, zur Herstellung eines Arzneimittels zur Behandlung von Lungenödemen.

## Claims

1. Medicament combinations which contain in addition to a compound **1** (6-hydroxy-8-{1-hydroxy-2-[2-(4-methoxy-phenyl)-1,1-dimethyl-ethylamino]-ethyl}-4H-benzo[1,4]oxazin-3-one) 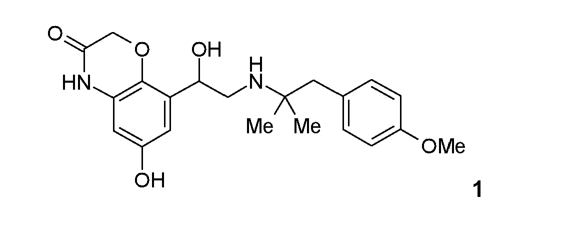 optionally in the form of the individual optical isomers, mixtures of the individual enantiomers or racemates, the acid addition salts with pharmacologically acceptable acids and optionally in the form of the solvates and/or hydrates;
as a further active substance **2** a tiotropium salt **2a** or ciclesonide **2b.**

2. Medicament combinations according to claim 1, which contain compound 1 in the form of the individual optical isomers, mixtures of the individual enantiomers or racemates.

3. Medicament combinations according to one of claims 1 or 2, which contain compound **1** in the form of the acid addition salts with pharmacologically acceptable acids and optionally in the form of the solvates and/or hydrates.

4. Medicament combinations according to one of claims 1 to 3, which contain the compound **2a** in the form of the bromide, optionally in the form of the solvates and/or hydrates.

5. Medicament combinations according to one of claims 1 to 4, **characterised in that** they contain a pharmaceutically acceptable carrier in addition to therapeutically effective amounts of **1** and **2.**

6. Medicament combinations according to one of claims 1 to 4, **characterised in that** they do not contain a pharmaceutically acceptable carrier in addition to therapeutically effective amounts of **1** and **2.**

7. Medicament combination according to one of claims 1 to 6, **characterised in that** it is present in the form of a formulation suitable for inhalation.

8. Medicament combination according to claim 7, **characterised in that** it is a formulation selected from among the inhalable powders, propellant-driven metered-dose aerosols and propellant-free inhalable solutions or suspensions.

9. Medicament combination according to claim 8, **characterised in that** the preparation is an inhalable powder which contains **1** and **2** in admixture with suitable physiologically acceptable excipients selected from among the monosaccharides, disaccharides, oligo- and polysaccharides, polyalcohols, salts, or mixtures of these excipients with one another.

10. Medicament combination according to claim 8, **characterised in that** the preparation is a propellant-drive inhalable aerosol which contains **1** and **2** in dissolved or dispersed form.

11. Medicament combination according to claim 10, **characterised in that** the inhalable aerosol contains as the propellant gas hydrocarbons such as n-propane, n-butane or isobutane or halohydrocarbons such as chlorinated and/or fluorinated derivatives of methane, ethane, propane, butane, cyclopropane or cyclobutane.

12. Medicament combination according to claim 11, **characterised in that** the propellant gas is TG11, TG12, TG134a, TG227 or mixtures thereof, preferably TG134a, TG227 or a mixture thereof.

13. Medicament combination according to claim 8, **characterised in that** the preparation is a propellant-free inhalable solution or suspension which contains as solvent water, ethanol or a mixture of water and ethanol.

14. Use of a medicament combination according to one of claims 1 to 11 for preparing a medicament for the treatment of inflammatory and obstructive respiratory complaints, for inhibiting premature labour in midwifery (tocolysis), for restoring sinus rhythm in the heart in atrioventricular block, for correcting bradycardic heart rhythm disorders (antiarrhythmic), for treating circulatory shock (vasodilatation and increasing the heart volume) as well as for the treatment of skin irritations and inflammation.

15. Use according to claim 14, for preparing a medicament for the treatment of respiratory complaints selected from the group comprising obstructive pulmonary diseases of various origins, pulmonary emphysema of various origins, restrictive pulmonary diseases, interstitial pulmonary diseases, cystic fibrosis, bronchitis of various origins, bronchiectasis, ARDS (adult respiratory distress syndrome) and all forms of pulmonary oedema.

16. Use according to claim 15, for preparing a medicament for the treatment of obstructive pulmonary diseases selected from among bronchial asthma, paediatric asthma, severe asthma, acute asthma attacks, chronic bronchitis and COPD (chronic obstructive pulmonary disease).

17. Use according to claim 15, for preparing a medicament for the treatment of pulmonary emphysema which has its origins in COPD or α1-proteinase inhibitor deficiency.

18. Use according to claim 15, for preparing a medicament for the treatment of restrictive pulmonary diseases selected from among allergic alveolitis, restrictive pulmonary diseases triggered by occupational noxious substances, such as asbestosis or silicosis, and restriction caused by lung tumours, such as for example lymphangiosis carcinomatosa, bronchoalveolar carcinoma and lymphomas.

19. Use according to claim 15, for preparing a medicament for the treatment of interstitial pulmonary diseases selected from among pneumonia caused by infections, such as for example infection by viruses, bacteria, fungi, protozoa, helminths or other pathogens, pneumonitis caused by various factors, such as for example aspiration and left heart insufficiency, radiation-induced pneumonitis or fibrosis, collagenoses, such as for example lupus erythematodes, systemic scleroderma or sarcoidosis, granulomatoses, such as for example Boeck's disease, idiopathic interstitial pneumonia or idiopathic pulmonary fibrosis (IPF).

20. Use according to claim 15, for preparing a medicament for the treatment of cystic fibrosis or mucoviscidosis.

21. Use according to claim 15, for preparing a medicament for the treatment of bronchitis, such as bronchitis caused by bacterial or viral infection, allergic bronchitis and toxic bronchitis.

22. Use according to claim 15, for preparing a medicament for the treatment of bronchiectasis.

23. Use according to claim 15, for preparing a medicament for the treatment of ARDS (adult respiratory distress syndrome).

24. Use according to claim 15, for preparing a medicament for the treatment of pulmonary oedema.

## Revendications

1. Combinaisons médicamenteuses qui contiennent, comme autre substance active 2, parallèlement à un composé de 1-(6-hydroxy-8-{1-hydroxy-2-[2-(4-méthoxyphényl)-1,1-diméthyléthylamino]-éthyl}-4H-benzo[1,4]oxazin-3-one, 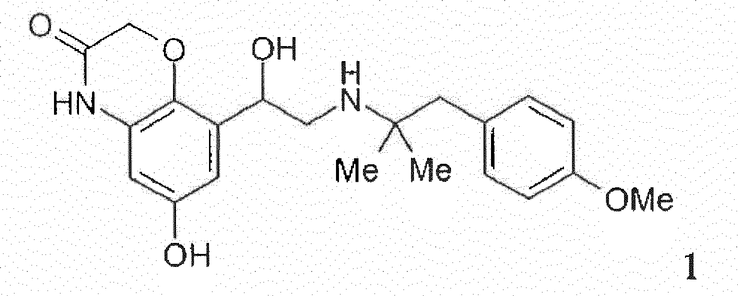 un sel de tiotropium 2a ou de ciclésonide 2b, éventuellement sous la forme des isomères optiques individuels, des mélanges d'énantiomères ou de racémates individuels, de sels d'addition acide avec des acides pharmacologiquement acceptable et éventuellement sous la forme de solvates et/ou hydratés.

2. Combinaisons médicamenteuses selon la revendication 1, qui contiennent un composé 1 sous la forme des isomères optiques individuels, de mélanges des énantiomères individuels ou de racémates.

3. Combinaisons médicamenteuses selon l'une des revendications 1 ou 2, qui contiennent le composé 1 sous la forme de sels d'addition acides avec des acides pharmacologiquement inoffensifs et éventuellement sous la forme de solvates et/ou hydrates.

4. Combinaisons médicamenteuses selon l'une des revendications 1 à 3 qui contiennent le composé 2a sous la forme de bromure, éventuellement sous la forme de solvates et/ou hydrates.

5. Combinaisons médicamenteuses selon l'une des revendications 1 à 4, **caractérisées en ce qu'**elles contiennent, outre des quantités thérapeutiquement efficaces des composés 1 et 2, un véhicule pharmaceutiquement acceptable.

6. Combinaisons médicamenteuses selon l'une des revendications 1 à 4, **caractérisées en ce que**, parallèlement à des quantités thérapeutiquement efficaces des composés 1 et 2, elles ne contiennent pas de véhicules pharmaceutiquement acceptables.

7. Combinaison médicamenteuse selon l'une des revendications 1 à 6, **caractérisée en ce qu'**elle se présente sous la forme d'une forme galénique appropriée pour l'inhalation.

8. Combinaison médicamenteuse selon la revendication 7, **caractérisée en ce qu'**il s'agit d'une forme galénique choisie dans le groupe comprenant les poudres pour inhalation, les aérosols doseurs contenant un gaz propulseur et les solutions ou suspensions pour Inhalation sans gaz propulseur.

9. Combinaison médicamenteuse selon la revendication 8, **caractérisée en ce que** la forme galénique est une poudre pour inhalation, qui contient les composés 1 et 2 en mélange avec des adjuvants physiologiquement inoffensifs appropriés choisis dans le groupe comprenant les monosaccharides, les disaccharides, les oligo- et polysaccharides, les poly-alcools, les sels ou des mélanges de ces adjuvants.

10. Combinaison médicamenteuse selon la revendication 8, **caractérisée en ce que** la forme galénique contient un aérosol pour inhalation contenant un gaz propulseur, qui contient les composés 1 et 2 sous une forme dissoute ou dispersé

11. Combinaison médicamenteuse selon la revendication 10, **caractérisée en ce que** l'aérosol pour inhalation contient comme gaz propulseur, des hydrocarbures tels que le n-propane, le n-butane et l'isobutane ou des hydrocarbures halogénés tels que des dérivés chlorés et/ou fluoré du méthane, de l'éthane, du propane, du butane, du cyclopropane ou du cyclobutane.

12. Combinaison médicamenteuse selon la revendication 11, **caractérisée en ce que** le gaz propulseur représente les TG11, TG12, TG134a, TG227 ou des mélanges de ceux-ci, de préférence les TG134a, TG227 ou un mélange de ceux-ci.

13. Combinaison médicamenteuse selon la revendication 8, **caractérisée en ce que** la forme galénique est une solution ou suspension pour inhalation sans gaz propulseur, qui contient comme solvant, de l'eau, de l'éthanol ou un mélange d'eau et d'éthanol.

14. Utilisation d'une combinaison médicamenteuse selon l'une des revendications 1 à 11, pour la production d'un médicament pour le traitement des maladies inflammatoires et obstructives des voies respiratoires, pour l'inhibition des contractions précoces en obstétrique (tocolyse), pour la restauration du rythme sinusal cardiaque dans le bloc auriculo-véntriculaire, pour la régularisation des troubles du rythme cardiaque de type bradycardie (anti-arythmique), pour le traitement du choc circulatoire (vasodilatation et augmentation du débit sanguin) et pour le traitement du prurit et des inflammations de la peau.

15. Utilisation selon la revendication 14, pour la production d'un médicament pour le traitement des maladies des voies respiratoires qui sont choisies dans le groupe comprenant les maladies pulmonaires obstructives d'origines diverses, les emphysèmes pulmonaires d'origines diverses, les maladies pulmonaires restrictives, les maladies pulmonaires interstitielles, la fibrose kystique, les bronchites d'origines diverses, les bronchiectasies, le SDRA (syndrome de détresse respiratoire de l'adulte) et toutes les formes d'oedèmes pulmonaires.

16. Utilisation selon la revendication 15, pour la production d'un médicament pour le traitement des maladies pulmonaires obstructives qui sont choisies dans le groupe comprenant l'asthme bronchique, l'asthme pédiatrique, l'asthme sévère, les crises d'asthme aiguës, la bronchite chronique et la BPCO (la bronchopneumopathie chronique obstructive).

17. Utilisation selon la revendication 15, pour la production d'un médicament pour le traitement des emphysèmes pulmonaires qui ont leur origine dans la BPCO ou dans un défaut d'inhibiteur de l'α1-protéinase.

18. Utilisation selon la revendication 15, pour la production d'un médicament pour le traitement des maladies pulmonaires restrictives, qui sont choisies dans le groupe comprenant l'alvéolite allergique, les maladies pulmonaires restrictives déclenchées par des polluants professionnels telles que l'asbestose ou la silicose et la restriction due à des tumeurs pulmonaires telles que par exemple, la lymphangiose carcinomateuse, le carcinome broncho-alvéolaire et les lymphomes.

19. Utilisation selon la revendication 15, pour la production d'un médicament pour le traitement des maladies pulmonaires interstitielles qui sont choisies dans le groupe comprenant les pneumonies d'origine infectieuse telles que par exemple, dues à une infection virale, bactérienne, une infection due à des champignons, des protozoaires, des vers ou d'autres agents pathogènes, les pneumonites d'origines diverses telles que par exemple, l'aspiration et l'insuffisance cardiaque gauche, la pneumonite ou la fibroses induite par des rayons, les collagénoses telles que par exemple, le lupus érythémateux, la sclérodermie ou la sarcoïdose systémique, les granulomatoses telles que par exemple, la maladie de Boeck, la pneumonie interstitielle idiopathique ou la fibrose pulmonaire idiopathique (IPF).

20. Utilisation selon la revendication 15, pour la production d'un médicament pour le traitement de la fibrose kystique ou de la mucoviscidose.

21. Utilisation selon la revendication 15, pour la production d'un médicament pour le traitement des bronchites telles que la bronchite due à une infection d'origine bactérienne ou virale, la bronchite allergique et la bronchite toxique.

22. Utilisation selon la revendication 15, pour la production d'un médicament pour le traitement des bronchiectasies.

23. Utilisation selon la revendication 15, pour la production d'un médicament pour le traitement du SDRA (syndrome de détresse respiratoire de l'adulte).

24. Utilisation selon la revendication 15, pour la production d'un médicament pour le traitement des oedèmes pulmonaires.
